(19) Europäisches Patentamt / European Patent Office / Office européen des brevets

(11) **EP 4 299 171 A1**

(12) **EUROPEAN PATENT APPLICATION**

(43) Date of publication:
**03.01.2024 Bulletin 2024/01**

(21) Application number: **22181202.7**

(22) Date of filing: **27.06.2022**

(51) International Patent Classification (IPC):
***B01J 14/00*** *(2006.01)* ***B01D 3/00*** *(2006.01)*
***C07C 67/00*** *(2006.01)* ***C07C 67/54*** *(2006.01)*

(52) Cooperative Patent Classification (CPC):
(C-Sets available)
**B01J 14/00; B01D 3/009; C07C 67/03;**
**C07C 67/08;** B01J 2219/00067; B01J 2219/00182

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Evonik Operations GmbH**
  **45128 Essen (DE)**
- **Evonik Corporation**
  **Parsippany, NJ 07054-0677 (US)**

(72) Inventors:
- **TRESKOW, Marcel**
  **64293 Darmstadt (DE)**
- **LACKEY, Kevin**
  **Daphne, AL 36526 (US)**
- **COONROD, Mark**
  **Cantonment, FL 32533 (US)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(54) **PROCESS FOR MAINTAINING A REACTOR FILLING LEVEL BY COMPENSATING CONVERSION-BASED VOLUME CONTRACTION OF A REACTION MIXTURE**

(57) The present invention discloses a method for maintaining a distillation reactor level during a reaction of a reaction mixture in the distillation reactor. The reaction mixture comprises at least one starting material and at least one product. The distillation reactor comprises a reboiler, a reaction chamber comprising the reaction mixture, a column with a column head, a feed line to the reaction chamber or to the column, a vapor transfer line, a condenser, a reflux tank, a reflux line, a distillate take off line, and a receiver vessel, wherein while the reaction is running at least a portion of at least one of the products is removed by distillate take off. The distillation reactor level is maintained by compensating a change in volume of the reaction mixture caused by the distillate take off and caused by the reaction of the reaction mixture.

**Figure 1:** Reactor system according to the invention, the reactor system comprising a reboiler (12), a reaction chamber (1), a column (2) with a column head (3), a vapor transfer line (4), a condenser (5), a reflux tank (6), a reflux line (7), a distillate take off line (8) with a mass flow meter (10), a receiver vessel (9), and a recycle line (11).

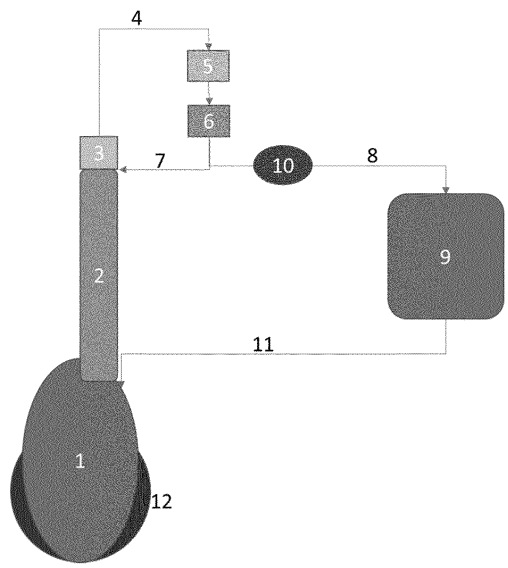


EP 4 299 171 A1

(52) Cooperative Patent Classification (CPC): (Cont.)

C-Sets
**C07C 67/03, C07C 69/54;**
**C07C 67/08, C07C 69/54**

## Description

### Field of the invention

The invention relates to the field of chemical products produced by starting materials in a reactor, whereby the level of the reactor filling is monitored and adjusted to make optimum use of its filling capacity and space-time yield.

### Background of the invention

During the process of product generation through starting materials, reactor filling is crucial for a well-balanced space-time yield. Too little reactor filling wastes available capacity and reduces therefore the space-time yield. Too much reactor filling can be critical to safety or at least a risk of safety concern. For this reason, there is always a safety buffer when calculating the volume of a batch to ensure sufficient air space is available. The air space is usually protected from complete filling by a level switch that indicates when a reactor reaches a filling level, which is too high.

The level within the reactor can be measured well and reliable with radar measurement, identifying the length of the air gab until which radar radiation is reflective by a radar responsive surface. However, it can be challenging for radar measurement to differentiate between foam and liquid level. It is not absolutely necessary that foam triggers a level switch to indicate an overfilled reactor as foam would collapse without further energy supply and thus the danger of overfilling the reactor would not be fulfilled. Therefore, radar measurement does not ensure optimal capacity use and space-time yield.

In addition, radar measurement has even more severe disadvantages especially with reactive contents in the reactor such as polymerizable substances. Over time these substances plug the radiation pathway, which causes false reading and force maintenance shutdown to replace the radar instrument. This caused radar measurement from basically been banned from reactive reactor contents.

Instead, a differential pressure measurement is used to calculate the level of a reactor filling with such critical material. Thereby, air is forced into the reactor at the bottom of the reactor and above the filling level at the top of the reactor. The pressure needed to force the air into the reactor at its bottom and its top is measured by pressure transmitters. The pressure difference with which the air is forced into the reactor at the bottom of the reactor and above the filling level at the top of the reactor is proportional to the weight of the liquid above the bottom pressure transmitter. By using the measured pressure difference and the density of the reactor contents or even mixed density of reactor contents, a reliable filling level calculation can be obtained.

Unfortunately, this level calculation is only as good as the input values used for the density of the substances in the reactor. If the density of the mixture changes significantly during the reaction process, the level calculation becomes incorrect as the reaction progresses. Even if the density has changed completely, the level is still assumed to be unchanged because the same amount of weight is above the pressure transmitter. In the worst case, this can lead to a situation where, if the density has decreased, the level volume has increased, and overfilling is imminent. In the opposite case, where the density has increased and the level volume has decreased, the reactor capacity is not fully utilized, resulting in a poor space-time yield.

Not only in general, but also in modern (trans)esterification reactions, like MMA transesterification reactions, there is a need to overcome the described problems as an optimal use of production capacity does not only lead to an increased space-time yield, but also to reduced production time and costs.

The object of this invention is to provide a method for calculating reactor filling that overcomes the aforementioned problems.

### **Brief** summary **of the invention**

The invention is directed to a method for maintaining a reaction chamber level of a reactor system during a reaction of a reaction mixture in the reaction chamber,

> wherein the reactor system comprises a reboiler, a reaction chamber comprising the reaction mixture, a column with a column head, a feed line to the reaction chamber or to the column, a vapor transfer line, a condenser, a reflux tank, a reflux line, and a distillate take off line, and a receiver vessel, and
> wherein the reaction mixture comprises at least one starting material and at least one product, wherein while the reaction is running at least a portion of at least one of the products is removed by distillate take off, wherein the reaction chamber level is maintained by compensating a change in volume of the reaction mixture caused by the distillate take off and caused by the reaction of the reaction mixture.

To this end, the present invention maintains reactor filling level by compensating conversion-based volume

contraction or expansion of a reaction mixture by calculating the change in volume along the reaction conversion to overcome the limitations described before and to prevent an overfilling or underfilling of the reactor. Each molecule of reacted starting material gives a change in density of the reaction mixture, as starting material is converted into the product and optional side product. If side products are distilled off during the reaction, this is taken into account by the calculation as well. The calculation of volume change due to density changes allows a precise estimation of reactor filling and possible replenishment of additional raw material in order to optimally use the reactor volume and reach an optimal space-time-yield.

## Description of the drawings

**Figure 1** illustrates a reactor system according to the invention, the reactor system comprises a reboiler (12), a reaction chamber (1), a column (2) with a column head (3), a vapor transfer line (4), a condenser (5), a reflux tank (6), a reflux line (7), a distillate take off line (8) with a mass flow meter (10), a receiver vessel (9), and a recycle line (11).

## Detailed description of the invention

Unless otherwise particularly defined herein, the related terms used in the present invention have the following definitions.

As used herein, the term "reactor system" refers to a reactor in which a chemical reaction of a reaction mixture can take place, and wherein the contents of the reactor can be subjected to a distillation process before the course of a chemical reaction and/or during the course of a chemical reaction and/or after the course of a chemical reaction or independently of a chemical reaction. The advantage of such a reactor is that it is not necessary to provide several chambers, one for a reaction to take place and one for the substance or substances to be distilled, thus saving material, time for a transfer of the substances and costs. The reactor system comprises at least a reboiler, a reaction chamber, a column with a column head, a feed line to the reaction chamber or to the column, a vapor transfer line, a condenser, a reflux tank, a reflux line, a distillate take off line, and a receiver vessel.

As used herein, the term "reaction chamber level" refers to a volume level or filling level in the reaction chamber of the reactor system.

As used herein, the term "reaction" refers, for example, to a (trans)esterification or (trans)esterification reaction.

As used herein, the term "(trans)esterification" or "(trans)esterification reaction" refers to both an esterification reaction and a transesterification reaction. Or it refers to either an esterification reaction or a transesterification reaction depending on the respective context.

As used herein, the term "(meth)acrylate" refers to both (meth)acrylic acid and (meth)acrylic acid ester. Furthermore, it refers to both methacrylate and acrylate. Or it refers to methacrylate or acrylate depending on the respective context. It also refers to methacrylic acid and methacrylic acid ester. For example, the term "a (meth)acrylate compound" refers to (meth)acrylic acid and a (meth)acrylic acid ester, e.g. an alkyl (meth)acrylate.

As used herein, the term "starting material" refers to raw material, initial material, educts, feedstock, reactants or initial reactants which can be used to undergo a chemical reaction, whereby the chemical reaction may result at least in a product or products, which can include side-products or by-products.

As used herein, the term "reboiler" refers to an apparatus for heating a liquid and/or converting a liquid to its vapor or, in other words, gaseous state.

As used herein, the term "reaction chamber" refers to a container in which a chemical reaction is carried out. There are a wide variety of chambers being referred to as such. For example, the sizes of reaction chambers range from micro reaction chambers, which hold a few microliters, to reaction chambers for a few milliliters, to chambers with a volume of numerous cubic meters. The most important characteristic of each reaction chamber is its resistance to the reaction conditions.

As used herein, the term "reaction mixture" refers to a mixture of substances that participate in a reaction. Such a reaction mixture can comprise or consist of:

- the starting material(s),
- the starting material(s) and possible additive(s) such as auxiliary substances, which can be, for example, catalysts or other reaction accelerators,
- the starting material(s) and product(s) including possible side-product(s),
- the starting material(s), additive(s) and product(s) including possible side-product(s),
- the product(s) including possible side-product(s) and the additive(s),
- the product(s) alone and additive(s),
- the product(s) including possible side-product(s), or
- the product(s) alone.

As used herein, the term "column" refers to an apparatus for the thermal separation of mixtures. To avoid heat loss, the column can be an insulated, preferably cylindrical, tube, which can be made in particular of steel, high-alloy stainless steels, glass or plastic. The height of the column body can mainly be dictated by the required quality of separation; the diameter by the volume flow of the mixture to be separated. The column can be placed between the reaction chamber and the distillation head. The number of individual distillations required for the same separation performance can also be referred to as the "theoretical plate number". At the surface of the column, the equilibrium between the liquid and gaseous phases can constantly be re-established by condensation and evaporation. As a result, the proportion of the low-boiling component continues to increase towards the top, while the higher-boiling component flows back into the reaction chamber, the sump. The size of the surface area of the column can be greatly increased in various ways by the design of trays, as in the Vigreux column, or by filling with packing or structured packing.

As used herein, the term "feed line" refers to supply lines which feed substances or substance mixtures to e.g. the reaction chamber or to the column.

As used herein, the term "condenser" refers to a unit in which the vapor produced during distillation, which can be composed of the various volatile components of the solution to be separated, can liquefy by cooling.

As used herein, the term "reflux tank" refers to a container into which condensed distillate flows, which then either flows back to the column and/or into the reaction chamber or is removed from the system.

As used herein, the term "reflux line" refers to a line which can feed distillate from the reflux tank back to the column or to the reaction chamber.

As used herein, the term "distillate take off line" refers to a line which can remove at least a portion of distillate from the reflux tank, or in general from the reactor system, or the distillation system.

The problem underlying the present invention is solved by a method for maintaining a reaction chamber level of a reactor system during a reaction of a reaction mixture in the reaction chamber, the reactor system comprises a reboiler, a reaction chamber comprising the reaction mixture, a column with a column head, a feed line to the reaction chamber or to the column, a vapor transfer line, a condenser, a reflux tank, a reflux line, a distillate take off line, and a receiver vessel, the reaction mixture comprises at least one starting material and at least one product, wherein while the reaction is running at least a portion of at least one of the products is removed by distillate take off, wherein the reaction chamber level is maintained by compensating a change in volume of the reaction mixture caused by the distillate take off, and caused by the reaction of the reaction mixture.

The method according to the invention has the advantage that it can indicate the level of the reaction chamber more accurately than the previous state of the art, in that the method includes volume contractions and expansions as well as volume inflows and outflows in the determination of the level of the reaction chamber. Overall, more accurate level determination has the advantage that additional reactants can be added preferably during the reaction to the reaction chamber to increase the time-space yield.

The reaction mixture may also comprise several starting materials and/or products. Furthermore, side products can be formed by the reaction, which can also be present in the reaction mixture.

Preferably, the change in volume of the reaction mixture is a volume contraction of the reaction mixture.

Preferably, the feed line is a first feed line, and the distillation reactor comprises a second feed line to the reaction chamber or to the column, and, optionally, further feed lines to the reaction chamber or to the column.

This has the advantage that substances and/or mixtures can be added to the reaction chamber and/or to the column via several feed lines. Substances and/or mixtures can be added simultaneously or successively and/or continuously or discontinuously via these feed lines. Different substances and/or mixtures can be added to the reaction chamber and/or to the column via the feed lines. Also, identical substances and/or mixtures can be added to the reaction chamber and/or to the column via the feed lines. Furthermore, identical substances and/or mixtures and different substances and/or mixtures can be added to the reaction chamber and/or to the column via the feed lines. These substances or mixtures can be, for example, the starting materials used for the reaction and/or additives such as reaction accelerators, reaction inhibitors, buffer, solvents, stabilizer, water, viscosity index improvers, thickeners, antioxidants, corrosion inhibitors, dispersants, high pressure additives, defoamers, catalysts or enzymes.

Preferably, the reaction mixture comprises two or more starting materials and two or more products. Further preferably, the two or more starting materials are a first educt, a second educt, and, optionally, one or more further compounds, and wherein the two or more products are a first product, a second product, and, optionally, one or more further products. Even more preferably, the one or more further compounds are selected from further educts, solvents, catalysts, and/or additives.

Preferably the compensating in this method is performed continuously, or discontinuously, e.g. in intervals, or occasionally upon need. This has the advantage of being able to respond flexibly to the demand in the reaction chamber and to control the feed and discharge so that the reaction chamber is optimally filled. Optimally filled means that the reaction chamber is filled in such a way that the space-time yield is optimized. Preferably, the reaction chamber is filled to the maximum filling level.

Preferably, in case of a volume contraction caused by the reaction of the reaction mixture the compensating

is performed by adding a higher amount of volume of at least one starting material to the reaction chamber than has been removed by the distillate take off. This has the advantage that the reaction volume in the reaction chamber can be kept as constant as possible, so that the space-time-yield is improved. Optimally, this process allows the reaction chamber filling level to remain at a maximum level. The distillate take off can be performed after condensation of the distillate during the reaction process. A measurement of the purity of the distillate can determine whether the distillate is withdrawn from the system or returned to the reaction chamber or to the column. The entire distillate can be withdrawn from the system, parts of the distillate can be withdrawn from the system, or no distillate can be withdrawn from the system. The amount of distillate withdrawn can vary depending on the stage of the reaction process.

Preferably, in case of a volume expansion caused by the reaction of the reaction mixture the compensating is performed adding a lower amount of volume of at least one starting material to the reaction chamber than has been removed by the distillate take off. This has the advantage that the reaction volume in the reaction chamber can also be kept as constant as possible under this reaction conditions, so that the space-time-yield is improved. Optimally, this process allows the reaction chamber filling level to remain at a maximum level. Here as well, the distillate take off can be performed after condensation of the distillate during the reaction process. Parts of the distillate can be withdrawn from the system, or no distillate can be withdrawn from the system. The amount of distillate withdrawn can vary depending on the stage of the reaction process.

The reaction in context of the method of the invention is preferably a (trans)esterification reaction for preparing an (meth)acrylate product. In case of a (trans)esterification reaction the reaction mixture may comprises an (meth)acrylate as first starting material and a first alcohol as second starting material which are converted by the (trans)esterification reaction into a second (meth)acrylate as a first product and a second alcohol (or water) as a second product. It is preferred that the second alcohol (or water) as a second product is removed by the distillate take off.

The above mentioned at least one starting material is preferably selected from the group consisting of alkyl (meth)acrylates, hydroxyalkyl (meth)acrylates, alkyl (meth)acrylate derivatives, and (meth)acrylic acid, and/or the at least one product is selected from the group consisting of alkyl (meth)acrylates, and alkyl (meth)acrylate derivatives.

Examples for starting materials in context of the invention are methyl methacrylate (MMA), hydroxyethyl methacrylate (HEMA), and methacrylic acid (MAA).

Examples for products in context of the invention are 2-ethylhexyl methacrylate (EHMA), ethylene glycol dimethylacrylate (EGDMA), and 2-ethylhexyl acrylate (EHAC), cetyl methacrylate (C16MA), magaryl methacrylate (C17MA), stearyl methacrylate (C18MA), myristyl methacrylate (C14MA), lauryl methacrylate (C12MA), benzyl methacrylate (BnMA), 1,4-butanediol dimethacrylate (1,4-BDDMA), cyclohexyl methacrylate (CHMA), tetrahydrofurfuryl methacrylate (THFMA), allyl methacrylate (AMA), glycerol formal methacrylate (CAS-Nr. 1620329-57-8), 1,3-butandiol dimethacrylate (1,3-BDDMA), trimethylolpropane trimethacrylate (TMPTMA), polyethylene glycol monomethyl ether methacrylate (MPEG methacrylate) having an average molecular weight from 250 g/mol up to 8000 g/mol), N-(2-methacryloyl oxyethyl) ethylene urea, and triethyleneglycol di(meth)acrylate.

In the method according to the invention the (meth)acrylate starting material may comprise methyl (meth)acrylate, and the product may comprise methanol, or the (meth)acrylate starting material may comprise ethyl (meth)acrylate, and the product may comprise ethanol, or the (meth)acrylate starting material may comprise n-butyl (meth)acrylate and the product may comprise butanol, or the (meth)acrylate starting material may comprise (meth)acrylic acid, and the product may comprise water, preferably the (meth)acrylate starting material comprises methyl (meth)acrylate, and the side product comprises methanol.

The reaction in the method of the invention preferably takes place in the presence of a catalyst.

In the method according to the invention the catalyst can be selected from titanium(IV) alcoholates, for example titanium(IV) tetraisopropanolate, titanium(IV) tetrabutanolate, titanium(IV) tetrakis(2-ethylhexanolate), or mixtures thereof; from zirconium acetylacetonate; or from strong basic compounds, for example alkali oxides, earth alkali oxides, alkali hydroxides, earth alkali hydroxides, alkali alkoxides, earth alkali alkoxides, alkali amides, and earth alkali amides, preferably wherein the strong basic catalyst comprises one or more compounds selected from the group consisting of calcium oxide (CaO), calcium hydroxide ($Ca(OH)_2$), lithium hydroxide (LiOH), sodium methanolate (NaOMe), lithium methanolate (LiOMe), lithium *tert*-butoxide (LiOt-Bu), lithium *iso*-propoxide (LiOiPr), lithium amide ($LiNH_2$), or mixtures thereof.

*Calculation method*

A reaction, here as an example a transesterification reaction, can be expressed as follows:

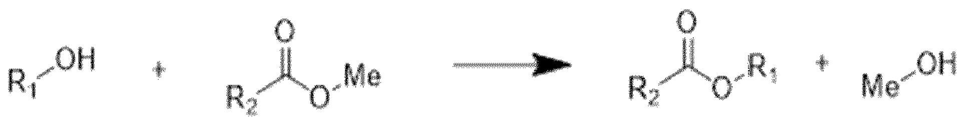

The side product methanol (MeOH) is removed during the transesterification reaction in the reaction chamber by distillation and does not remain within the reactor. Therefore, only the volume of the raw products, raw alcohol, and methyl methacrylate (MMA), are to compare with the products density, on a stoichiometric level.

In general, each compound has a specific molecular mass and a specific density. The reaction stoichiometry explains how many molecules of each raw material are converted to product. The calculation to estimate the density change uses a direct comparison of the volume of starting materials versus the volume of the product. Removed side products are not included in the calculation as they do not remain in the reactor. The volume (V) of each starting material is given by mass (m) and density (d), whereas the mass is available by stoichiometry and molecular mass (M). The sum of all partial volumes of raw materials is $V_{raws}$. The combined sum of all products that remain in the reactor is the volume $V_{prod}$. The difference of both volumes is the conversion dependent level shrink $V_{shrink}$ of the reactor.

$$V_{shrink} = V_{raws} - V_{prod}$$

$V_{shrink}$ explains the total volume difference between the stoichiometric raw materials and product. This is not equal to the total reactor volume shrink if the total raw volumes are used as these includes the atoms for the side product (raw side product) e.g. MeOH, which is not included in the $V_{prod}$, as it is removed from the reaction mixture. Each molecule side product e.g. MeOH, removed of the reactor can be replaced with more raw material e.g. MMA and in addition the volume gained by conversion can also be filled by raw material. The loss of volume per molecule side product ($V_{nshrink}$) can be calculated as follows:

$$V_{shrink}/n_{raw\ side\ product} = Vn_{shrink}$$

The volume change ($V_{change}$) per molecule side product removed ($n_{side\ product\ removed}$) can be calculated as follows:

$$Vn_{shrink} \times n_{side\ product\ removed} = V_{change}$$

As not molecules are measured, but mass, we can transform: n = m/M, wherein M is the molar mass and m is mass. The amount of substance is represented by the variable n.

$V_{change}$ per mass side product removed ($m_{side\ product\ removed}$) can be calculated as follows:

$$Vn_{shrink} \times \frac{m_{side\ product\ removed}}{M_{side\ product}} = V_{change}$$

This gives a direct relation between the side product, e.g. MeOH, taken off and the additional volume that can be added. With the density of the raw material a factor can be calculated to convert $m_{side}$ product removed to additional raw material added. Since *density* = $^{mass}/_{volume}$, the mass of raw material ($m_{raw\ material}$) is available by getting the density of the raw material ($d_{raw\ material}$) involved:

$$V_{change} \times d_{raw\ material} = m_{raw\ material}$$

The relation between side product take off and raw material addition to the final calculation how much more raw material to add per mass side product taken off is calculated as follows:

$$d_{raw\ material} \times Vn_{shrink} \times \frac{m_{side\ product\ removed}}{M_{side\ product}} = m_{raw\ material\ to\ add}$$

The mass of raw material to add is herein $m_{raw\ material\ to\ add}$.

Each mass of raw product e.g. MMA, which might be in the take off stream is replaced with equal amounts trickle feed, to compensate non reaction losses. If raw material is in the take off stream can be determined for example by density calculation of the take of stream or by chromatography or other measurement methods.

If for some reason the volume is increased during the reaction, since the product as a lower density than the raw material mixture, the same calculation as presented above gives the amount to be removed from the reaction mixture in the reactor to prevent reactor overfilling. The factor, then, will be smaller than one.

Further advantages, details and features of the invention will be apparent below from the examples explained.

**Examples**

*Example 1: Transesterification of 2-ethylhexanol and MMA*

In this example 1, 1000 kg 2-ethylhexanol (EHOH) and 768.8 kg methyl methacrylate (MMA) are transesterified to give 2-ethylhexyl methacrylate (EHMA).

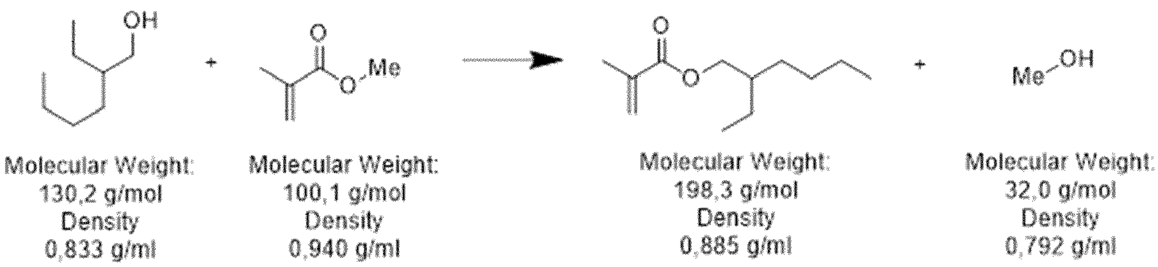


Scheme 1: Transesterification of EHOH and MMA

The volume of EHOH is:

$$V_{EHOH} = \frac{1000\ kg}{0.833\ \frac{kg}{l}} = 1200\ l$$

The amount of EHOH is:

$$n_{EHOH} = \frac{1000\ kg}{130.2\ \frac{kg}{kmol}} = 7.68\ kmol$$

As the amount of EHOH equals the amount of MMA

$$n_{EHOH} = n_{MMA} = 7.68\ kmol,$$

the amount of MMA is as follows:

$$m_{MMA} = 100.1\ \frac{kg}{kmol} \times 7.68\ kmol = 768.8\ kg$$

The volume of MMA therefore is:

$$V_{MMA} = \frac{768.8\ kg}{0.94\ \frac{kg}{l}} = 817.9\ l$$

And the volume of raw materials is:

$$V_{raws} = 1200\ l + 817.9\ l = 2018.4\ l$$

As $n_{EHOH} = n_{EHMA}$ the mass of EHMA is

$$m_{EHMA} = 7.68\ kmol \times 198.3\ \frac{kg}{mol} = 1522.9\ kg,$$

the volume of the product can be calculated as follows:

$$V_{prod} = \frac{1522.9\ kg}{0.89\ \frac{kg}{l}} = 1711\ l$$

Therefore, the conversion dependent level shrink of the reactor is:

$$V_{shrink} = 2018.4\ l - 1711\ l = 307.4\ l$$

The loss of volume per molecule side product MeOH is:

$$Vn_{shrink} = \frac{307.4\ l}{7.68\ kmol} = 40\ \frac{l}{kmol}$$

The raw material to add is:

$$d_{raw\ material} \times Vn_{shrink} \times \frac{m_{MeOH\ removed}}{M_{MeOH}} = m_{raw\ material\ to\ add}$$

For 1 kg MeOH this is:

$$0.94\ \frac{kg}{l} \times 40 \frac{l}{kmol} \times \frac{1\ kg_{MeOH\ removed}}{32 \frac{kg}{kmol}} = 1.17\ kg\ ,$$

which means each 1 kg MeOH shall be replaced with 1.17 kg MMA amount. This means an amount of MeOH is to be replaced with 1.17 times the MMA amount.

If an azeotrope of MMA and MeOH occurs during distillation procedure and the azeotrope has 80% purity 1 kg consist of 200 g MMA and 800 g MeOH. The total amount of trickle feed added back is therefore:

$$200\ g + 800\ g \times 1.17 = 200\ g + 940\ g = 1140\ g$$

Therefore, 1140 g MMA should be fed to the reactor chamber in this case to maintain the reactor filling level.

*Example 2: Transesterification of hydroxyethyl methacrylate (HEMA) and MMA*

In this example 2, 1000 kg HEMA and 769.2 kg MMA are transesterified to give ethylene glycol dimethylacrylate (EGDMA).

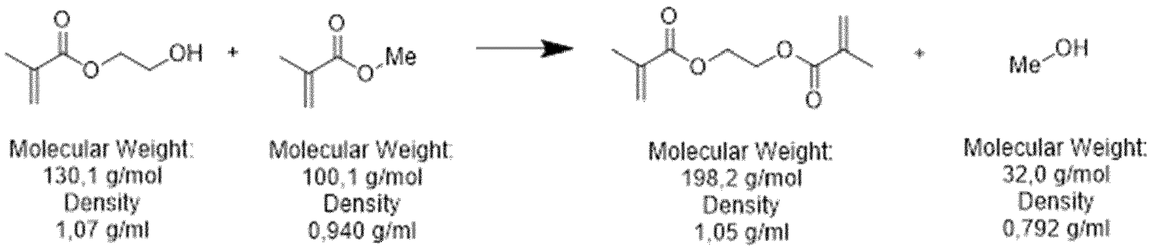


Scheme 2: Transesterification of HEMA and MMA

The volume of HEMA is:

$$V_{HEMA} = \frac{1000\ kg}{1.07\ \frac{kg}{l}} = 934.6\ l$$

The amount of HEMA is:

$$n_{HEMA} = \frac{1000\ kg}{130.14\ \frac{kg}{kmol}} = 7.684\ kmol$$

As the amount of HEMA equals the amount of MMA

$$n_{HEMA} = n_{MMA} = 7.684\ kmol,$$

the amount of MMA is as follows:

$$m_{MMA} = 100.1\ \frac{kg}{kmol} \times 7.684\ kmol = 769{,}2\ kg$$

The volume of MMA therefore is:

$$V_{MMA=} \frac{769.2\ kg}{0.94\ \frac{kg}{l}} = 818.2\ l$$

And the volume of raw materials is:

$$V_{raws} = 934.6\ l + 818.2\ l = 1752.8\ l$$

As $n_{HEMA}$ = $n_{EHMA}$ the mass of EHMA is

$$m_{EHMA} = 7.684\ kmol \times 198.218\ \frac{kg}{mol} = 1523.1\ kg,$$

the volume of the product can be calculated as follows:

$$V_{prod} = \frac{1523.1\,kg}{1.051\,\frac{kg}{l}} = 1449.2\,l$$

Therefore, the conversion dependent level shrink of the reactor is:

$$V_{shrink} = 1752.8\,l - 1449.2\,l = 303.6\,l$$

The loss of volume per molecule side product MeOH is:

$$Vn_{shrink} = \frac{303.6\,l}{7.684\,kmol} = 39.5\,\frac{l}{kmol}$$

The raw material to add is:

$$d_{raw\ material} \times Vn_{shrink} \times \frac{m_{MeOH\ removed}}{M_{MeOH}} = m_{raw\ material\ to\ add}$$

For 1 kg MeOH this is:

$$0.94\,\frac{kg}{l} \times 39.5\,\frac{l}{kmol} \times \frac{1\,kg_{MeOH\ removed}}{32\frac{kg}{kmol}} = 1.16\,kg\ ,$$

which means each 1kg MeOH shall be replaced with 1.16 kg MMA amount. This means an amount of MeOH is to be replaced with 1.16 time the MMA amount.

If an azeotrope of MMA and MeOH occurs during distillation procedure and the azeotrope has 80% purity 1 kg consist of 200 g MMA and 800 g MeOH. The total amount of trickle feed added back is therefore:

$$200\,g + 800\,g \times 1.16 = 200\,g + 928\,g = 1128\,g$$

Therefore, 1128 g MMA should be fed to the reactor chamber in this case to maintain the reactor filling level.

*Example 3: Esterification of methacrylic acid (MAA) and 1-octadecanol alcohol ($C_{18}OH$)*

In this example 3, 1000 kg $C_{18}OH$ and 254.3 kg MAA are transesterified to give stearyl methacrylate ($C_{18}MA$).

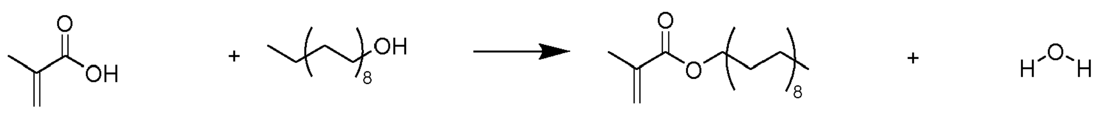

Molecular Weight: 86,1 g/mol Density 1,02 g/ml

Molecular Weight: 270,5 g/mol Density 0,83 g/ml

Molecular Weight: 338,6 g/mol Density 0,864 g/ml

Molecular Weight: 18,0 g/mol Density 0,997 g/ml

Scheme 3: Esterification of $C_{18}OH$ and MAA

The volume of $C_{18}OH$ is:

$$V_{\mathrm{C18OH}} = \frac{1000\ kg}{0.83\ \frac{kg}{l}} = 1205\ l$$

The amount of HEMA is:

$$n_{\mathrm{C18OH}} = \frac{1000\ kg}{338.6\ \frac{kg}{kmol}} = 2.953\ kmol$$

As the amount of $C_{18}OH$ equals the amount of MAA

$$n_{\mathrm{C18OH}} = n_{MAS} = 2.953\ kmol,$$

the amount of MAA is as follows:

$$m_{MAS} = 86.1\ \frac{kg}{kmol} \times 2.953\ kmol = 254.3\ kg$$

The volume of MAA therefore is:

$$V_{MAS=} \frac{254.3\ kg}{1.02\ \frac{kg}{l}} = 249.3\ l$$

And the volume of raw materials is:

$$V_{raws} = 249.3\ l + 1205\ l = 1454.3\ l$$

As $n_{C18OH} = n_{C18MA}$ the mass of $C_{18}MA$ is

$$m_{\mathrm{C18MA}} = 2.953\ kmol \times 338.6\ \frac{kg}{mol} = 999.9\ kg,$$

the volume of the product can be calculated as follows:

$$V_{prod} = \frac{999.9\ kg}{0.864\ \frac{kg}{l}} = 1157.3\ l$$

Therefore, the conversion dependent level shrink of the reactor is:

$$V_{shrink} = 1454.3\ l - 1157.3\ l = 297\ l$$

The loss of volume per molecule side product water ($H_2O$) is:

$$Vn_{shrink} = \frac{297\ l}{2.953\ kmol} = 100.57\ \frac{l}{kmol}$$

The raw material to add is:

$$d_{raw\ material} \times Vn_{shrink} \times \frac{m_{H2O\ removed}}{M_{H2O}} = m_{raw\ material\ to\ add}$$

For 1 kg $H_2O$ this is: $1.02\ \frac{kg}{l} \times 100.57 \frac{l}{kmol} \times \frac{1\ kg_{H2O\ removed}}{18 \frac{kg}{kmol}} = 5.70\ kg$, which means each 1kg $H_2O$ shall be replaced with 5.7 kg MAA amount. This means an amount of water is to be replaced with 5.70 time the MAA amount.

If an azeotrope of MAA and $H_2O$ occurs during distillation procedure and the azeotrope has 80% purity 1 kg consist of 200 g MAA and 800 g $H_2O$. The total amount of trickle feed added back is therefore:

$$200\ g + 800\ g \times 5.70 = 200\ g + 4560\ g = 4760\ g$$

Therefore, 4760 g MMA should be feed to the reactor chamber in this case to maintain the reactor filling level.

*Example 4: Transesterification of methyl acetate (MAC) and 2-Ethvlhexanol*

In this example 4, 1000 kg EHOH and 569.1 kg MAC are transesterified to give 2-ethylhexyl acetate (EHAC).

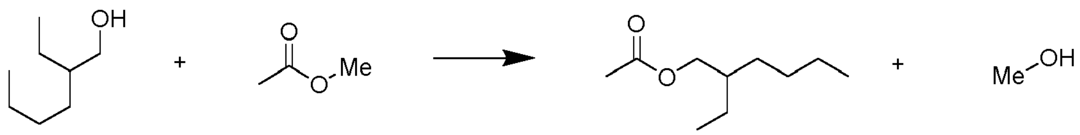

Molecular Weight: 130,2 g/mol Density 0,833 g/ml

Molecular Weight: 74,1 g/mol Density 0,932 g/ml

Molecular Weight: 172,3 g/mol Density 0,88 g/ml

Molecular Weight: 32,0 g/mol Density 0,792 g/ml

Scheme 4: Transesterification of EHOH and MAC

The volume of EHOH is:

$$V_{\mathrm{EHOH}} = \frac{1000\ kg}{0.833\ \frac{kg}{l}} = 1200\ l$$

The amount of EHOH is:

$$n_{\mathrm{EHOH}} = \frac{1000\ kg}{130.2\ \frac{kg}{kmol}} = 7.68\ kmol$$

As the amount of EHOH equals the amount of MAC

$$n_{\mathrm{EHOH}} = n_{\mathrm{MAC}} = 7.68\ \ kmol,$$

the amount of MAC is as follows:

$$m_{MAC} = 74.1\ \frac{kg}{kmol} \times 7.68 kmol = 569.1 kg$$

The volume of MAC therefore is:

$$V_{MAC=} \frac{569.1\ kg}{1.02\ \frac{kg}{l}} = 557.9\ l$$

And the volume of raw materials is:

$$V_{raws} = 1200\ l + 557.9\ l = 1757.9\ l$$

As $n_{EHOH} = n_{EHAC}$ the mass of EHAC is

$$m_{\mathrm{EHAC}} = 7.68\ kmol \times 172.3\ \frac{kg}{mol} = 1323.3\ kg,$$

the volume of the product can be calculated as follows:

$$V_{prod} = \frac{1323.3\ kg}{0.88\ \frac{kg}{l}} = 1503.8\ \mathrm{l}$$

Therefore, the conversion dependent level shrink of the reactor is:

$$V_{shrink} = 1757.9\ l - 1503.8\ l = 254.15\ l$$

The loss of volume per molecule side product water ($H_2O$) is:

$$Vn_{shrink} = \frac{254.15\ \ l}{7.68\ kmol} = 33{,}1 \frac{l}{kmol}$$

The raw material to add is:

$$d_{raw\ material} \times Vn_{shrink} \times \frac{m_{MeOH\ removed}}{M_{MeOH}} = m_{raw\ material\ to\ add}$$

For 1 kg MeOH this is: $0.932\ \frac{kg}{l} \times 33.1 \frac{l}{kmol} \times \frac{1\ kg_{H2O\ removed}}{32 \frac{kg}{kmol}} = 0{,}964\ kg$, which means each 1kg MeOH shall be replaced with 0.964kg MAC amount. This means an amount of MeOH is to be replaced with 0.964 time the MAC amount.

If an azeotrope of MAC and Methanol occurs during distillation procedure and the azeotrope has 20% purity 1 kg consist of 800 g MAC and 200 g MeOH. The total amount of trickle feed added back is therefore:

$$800\ g + 200\ g \times 0.964 = 800\ g + 193\ g = 993\ g$$

Therefore, 993 g MAC should be feed to the reactor chamber in this case to maintain the reactor filling level.

## Claims

1. Method for maintaining a reaction chamber level of a reactor system during a reaction of a reaction mixture in the reaction chamber,

   wherein the reactor system comprises a reboiler (12), a reaction chamber (1) comprising the reaction mixture, a column (2) with a column head (3), a feed line to the reaction chamber or to the column, a vapor transfer line (4), a condenser (5), a reflux tank (6), a reflux line (7), a distillate take off line (8), and a receiver vessel (9), and wherein the reaction mixture comprises at least one starting material and at least one product, wherein while the reaction is running at least a portion of at least one of the products is removed by distillate take off, wherein the reaction chamber level is maintained by compensating a change in volume of the reaction mixture caused by the distillate take off, and caused by the reaction of the reaction mixture.

2. Method according to claim 1, wherein the change in volume of the reaction mixture is a volume contraction of the reaction mixture.

3. Method according to claim 1 or 2, wherein the feed line is a first feed line, and wherein the reactor system comprises a second feed line to the reaction chamber or to the column, and, optionally, further feed lines to the reaction chamber or to the column.

4. Method according to one or more of the preceding claims, wherein the reaction mixture comprises two or more starting materials and two or more products.

5. Method according to claim 4, wherein the two or more starting materials are a first educt, a second educt, and, optionally, one or more further compounds, and wherein the two or more products are a first product, a second product, and, optionally, one or more further products.

6. Method according to claim 5, wherein the one or more further compounds are selected from further educts, solvents, catalysts, and/or additives.

7. Method according to one or more of the preceding claims, wherein the compensating is performed continuously, in intervals, or occasionally upon need.

8. Method according to one or more of the preceding claims, wherein

   a) in case of a volume contraction caused by the reaction of the reaction mixture the compensating is performed by adding a higher amount of volume of at least one starting material to the reaction chamber than has been removed by the distillate take off, or
   b) in case of a volume expansion caused by the reaction of the reaction mixture the compensating is performed adding a lower amount of volume of at least one starting material to the reaction chamber than has been removed by the distillate take off.

9. Method according to one or more of the preceding claims, wherein the reaction is a (trans)esterification reaction for preparing a (meth)acrylate product.

10. Method according to claim 9, wherein the reaction mixture comprises a (meth)acrylate as first starting material and a first alcohol as second starting material which are converted by the (trans)esterification reaction into a second (meth)acrylate as a first product and a second alcohol or water as a second product.

11. Method according to one or more of the preceding claims, wherein at least one of the starting materials is selected from the group consisting of alkyl (meth)acrylates, hydroxyalkyl (meth)acrylates, alkyl (meth)acrylate derivatives, and (meth)acrylic acid, and/or wherein the at least one of the products is selected from the group consisting of alkyl (meth)acrylates, and alkyl (meth)acrylate derivatives.

12. Method according to one or more of the preceding claims, wherein one of the starting materials is selected from the

group consisting of methyl methacrylate (MMA), hydroxyethyl methacrylate (HEMA), and methacrylic acid (MAA).

**13.** Method according to one or more of the preceding claims, wherein one of the products is selected from the group consisting of 2-ethylhexyl methacrylate, ethylene glycol dimethylacrylate, and 2-ethylhexyl acrylate, cetyl methacrylate, magaryl methacrylate, stearyl methacrylate, myristyl methacrylate, lauryl methacrylat, benzyl methacrylat, 1,4-butanedioldimethacrylat, cyclohexylmethacrylate, tetrahydrofurfuryl methacrylate, allyl methacrylate, glycerol formal methacrylate, 1,3-butandioldimethacrylat, trimethylolpropane trimethacrylat, polyethylene glycol monomethyl ether methacrylate (having an average molecular weight from 250 g/mol up to 8000 g/mol), N-(2-methacryloyl oxyethyl) ethylene urea, and triethylene glycol di(meth)acrylate.

**14.** Method according to one or more of the preceding claims, wherein the reaction takes place in the presence of a catalyst.

**Figure 1:** Reactor system according to the invention, the reactor system comprising a reboiler (12), a reaction chamber (1), a column (2) with a column head (3), a vapor transfer line (4), a condenser (5), a reflux tank (6), a reflux line (7), a distillate take off line (8) with a mass flow meter (10), a receiver vessel (9), and a recycle line (11).

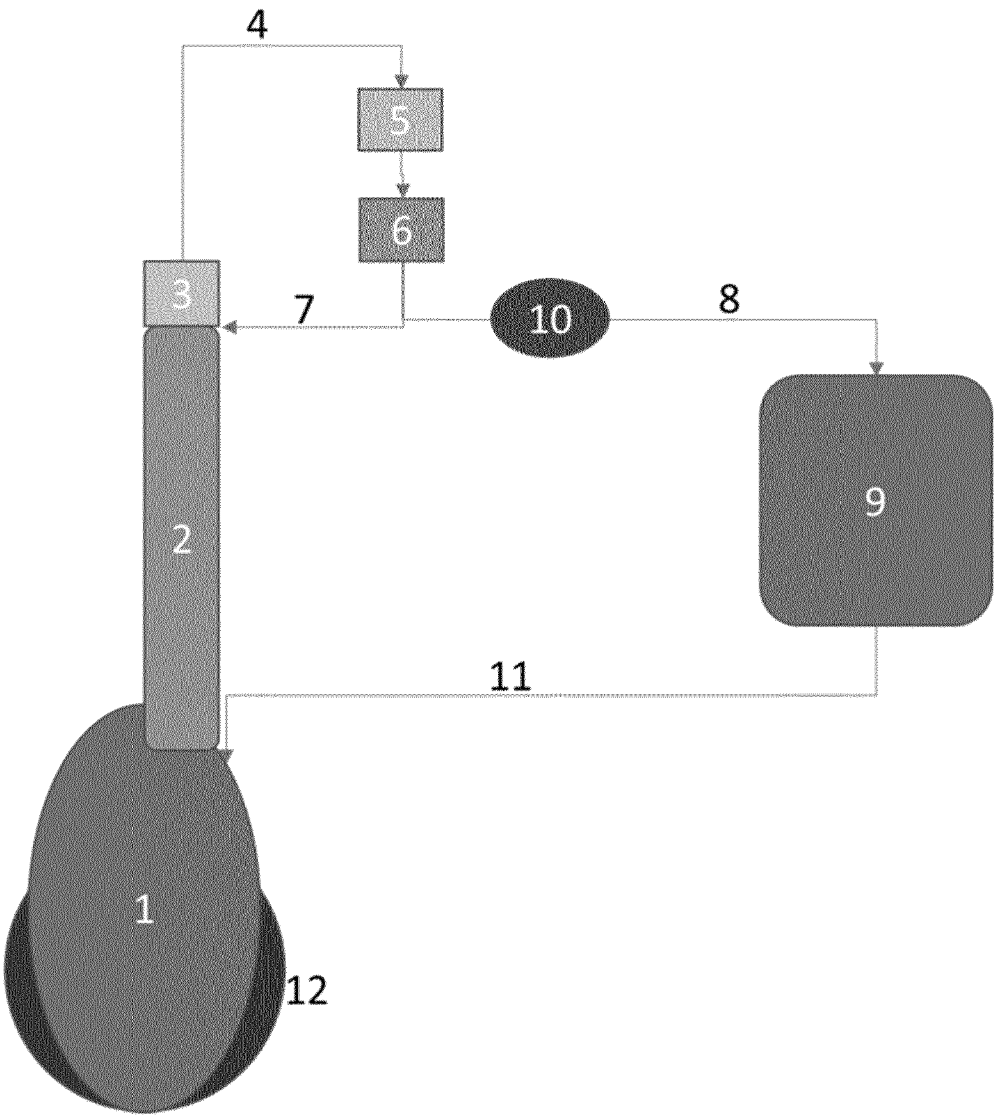

Europäisches Patentamt
European Patent Office
Office européen des brevets

# EUROPEAN SEARCH REPORT

Application Number
EP 22 18 1202

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LARSSON T. ET AL: "Control Structure Selection for Reactor, Separator, and Recycle Processes", INDUSTRIAL & ENGINEERING CHEMISTRY RESEARCH, 19 February 2003 (2003-02-19), pages 1225-1234, XP093005651, ISSN: 0888-5885, DOI: 10.1021/ie0200860 Retrieved from the Internet: URL:https://pubs.acs.org/doi/pdf/10.1021/ie0200860 [retrieved on 2022-12-06] | 1-8 | INV. B01J14/00 B01D3/00 C07C67/00 C07C67/54 |
| Y | * figure 4 * | 9-14 | |
| X | Mcmillan Greg: "Achieving Tight Control of Single Phase Liquid Reactors", , 28 September 2020 (2020-09-28), pages 1-10, XP093005659, Retrieved from the Internet: URL:https://blog.isa.org/tight-control-single-phase-indutrial-liquid-reactors [retrieved on 2022-12-07] * page 3 * | 1-8 | |
| X | CN 207 169 670 U (CHANGCHUN INST APPLIED CHEMISTRY CAS) 3 April 2018 (2018-04-03) | 1,2,4-8 | |
| Y | * figure 1 * | 9-14 | |
| A | CN 105 327 521 A (BEFAR GROUP CO LTD) 17 February 2016 (2016-02-17) * figure 1 * | 1-7 | |
| Y | US 2010/240923 A1 (HAERING DIETMAR [DE] ET AL) 23 September 2010 (2010-09-23) * paragraphs [[0060]], [[0061]], [[0058]], [[0005]], [[0075].] * | 9-14 | |

TECHNICAL FIELDS SEARCHED (IPC)

B01J
F17C
B01D
C07C

The present search report has been drawn up for all claims

1

| Place of search | Date of completion of the search | Examiner |
|---|---|---|
| Munich | 7 December 2022 | Pasanisi, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 18 1202

This annex lists the patent family members relating to the patent documents cited in the above-mentioned European search report. The members are as contained in the European Patent Office EDP file on
The European Patent Office is in no way liable for these particulars which are merely given for the purpose of information.

07-12-2022

| Patent document cited in search report | | Publication date | Patent family member(s) | | | Publication date |
|---|---|---|---|---|---|---|
| CN 207169670 | U | 03-04-2018 | NONE | | | |
| CN 105327521 | A | 17-02-2016 | NONE | | | |
| US 2010240923 | A1 | 23-09-2010 | EP | 2225384 | A1 | 08-09-2010 |
| | | | US | 2010240923 | A1 | 23-09-2010 |
| | | | WO | 2009068098 | A1 | 04-06-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS,* 1620329-57-8